# EUROPÄISCHE PATENTANMELDUNG

(11) **EP 1 035 106 A1**
(43) Veröffentlichungstag der Anmeldung: **13.09.2000**
(21) Anmeldenummer: 00102220.1
(22) Anmeldetag: 10.02.2000
(51) Int. Cl.: C07C 209/26

(54) **Verfahren zur Herstellung von Aminen**

(30) Priorität: 12.03.1999 DE 19910960
(71) Anmelder: BASF AKTIENGESELLSCHAFT, 67056 Ludwigshafen (DE)
(72) Erfinder: Funke, Frank, Dr., 67117 Limburgerhof (DE); Wulff-Döring, Joachim, Dr., 67227 Frankenthal (DE); Schulz, Gerhard, Dr., 67079 Ludwigshafen (DE); Siegel, Wolfgang, Dr., 67117 Limburgerhof (DE); Kramer, Andreas, Dr., 67251 Freinsheim (DE); Melder, Johann-Peter, Dr., 67459 Böhl-Iggelheim (DE); Höhn, Arthur, Dr., 67281 Kirchheim (DE); Buskens, Philipp, Dr., 23204 Hoogstraaten (BE); Reif, Wolfgang, Dr., 67227 Frankenthal (DE); Nouwen, Jan, Dr., 67157 Wachenheim (DE)

(57) **Zusammenfassung**

Herstellung von Aminen durch Umsetzung von Aldehyden oder Ketonen bei erhöhter Temperatur und erhöhtem Druck mit Stickstoffverbindungen, ausgewählt aus der Gruppe Ammoniak, primäre und sekundäre Amine, und mit Wasserstoff in Gegenwart eines Katalysators enthaltend Kupfer, wobei die katalytisch aktive Masse des Katalysators vor der Reduktion mit Wasserstoff
20 bis 85 Gew.-% sauerstoffhaltige Verbindungen des Zirkoniums, berechnet als ZrO₂,
1 bis 30 Gew.-% sauerstoffhaltige Verbindungen des Kupfers, berechnet als CuO,
14 bis 70 Gew.-% sauerstoffhaltige Verbindungen des Nickels, berechnet als NiO,
0 bis 5 Gew.-% sauerstoffhaltige Verbindungen des Molybdäns, berechnet als MoO₃, und 0 bis 10 Gew.-% sauerstoffhaltige Verbindungen des Aluminiums, berechnet als Al₂O₃, enthält.

## Beschreibung

Die vorliegende Erfindung betrifft ein Verfahren zur Herstellung von Aminen durch Umsetzung von Aldehyden oder Ketonen bei erhöhter Temperatur und erhöhtem Druck mit Stickstoffverbindungen, ausgewählt aus der Gruppe Ammoniak, primäre und sekundäre Amine, und mit Wasserstoff in Gegenwart eines Katalysators enthaltend Kupfer.

Aus der EP-A-514 692 sind Kupfer-, Nickel- und/oder Kobalt-, Zirkonium- und/oder Aluminiumoxid enthaltende Katalysatoren zur katalytischen Aminierung von Alkoholen in der Gasphase mit Ammoniak oder primären Aminen und Wasserstoff bekannt.
Diese Patentanmeldung lehrt, dass bei diesen Katalysatoren das Atomverhältnis von Nickel zu Kupfer 0,1 bis 1,0, bevorzugt 0,2 bis 0,5 (vergl. loc. cit.: Beispiel 1) betragen muß, da ansonsten bei der Aminierung von Alkoholen in erhöhtem Maße ausbeutemindernde Nebenprodukte auftreten (loc. cit.: Beispiele 6 und 12).
Als Träger wird bevorzugt Aluminiumoxid (loc. cit.: Beispiele 1 bis 5 und 7 bis 11) verwendet.

Aus der EP-A-382 049 sind Katalysatoren, die sauerstoffhaltige Zirkonium-, Kupfer-, Kobalt- und Nickelverbindungen enthalten, und Verfahren zur hydrierenden Aminierung von Alkoholen bekannt.
Der bevorzugte Zirkoniumoxidgehalt dieser Katalysatoren beträgt 70 bis 80 Gew.-% (loc. cit.: Seite 2, letzter Absatz; Seite 3, 3. Absatz; Beispiele). Diese Katalysatoren zeichnen sich zwar durch eine gute Aktivität und Selektivität aus, besitzen allerdings verbesserungswürdige Standzeiten.

Aus der EP-A-696 572 und der EP-A-697 395 sind Nickel-, Kupfer-, Zirkonium- und Molybdänoxid enthaltende Katalysatoren zur katalytischen Aminierung von Alkoholen mit Stickstoffverbindungen in Gegenwart von Wasserstoff bekannt.

Die älteren deutschen Anmeldungen Nr. 19742911.4 vom 29.09.97 und 19826396.1 vom 12.06.98 betreffen Verfahren zur Herstellung von Aminen durch Umsetzung von primären oder sekundären Alkoholen mit Stickstoffverbindungen, ausgewählt aus der Gruppe Ammoniak, primäre und sekundäre Amine, bei Temperaturen von 80 bis 250°C und Drücken von 0,1 bis 40 MPa mit Wasserstoff in Gegenwart von Katalysatoren enthaltend Zirkonium, Kupfer und Nickel.

DE-A-28 38 184 beschreibt ein Verfahren zur Herstellung tertiärer Amine durch Umsetzung sekundärer Amine mit Alkoholen oder Carbonylverbindungen unter hydrierenden Bedingungen in der Gasphase, indem man die Umsetzung an einem Kupferkatalysator vornimmt, der durch thermische Zersetzung und Reduktion eines basischen Kupfer-Aluminium-Carbonats erhalten worden ist.

Der vorliegenden Erfindung lag die Aufgabe zugrunde, unter Abhilfe der Nachteile des Stands der Technik die Wirtschaftlichkeit bisheriger Verfahren zur hydrierenden Aminierung von Aldehyden und Ketonen zu verbessern. Es sollten Katalysatoren gefunden werden, die technisch in einfacher Weise herzustellen sind und die es erlauben, die hydrierende Aminierung von Aldehyden und Ketonen mit hohem Umsatz, guter Ausbeute, Selektivität und Katalysatorstandzeit durchzuführen. Die Katalysatoren sollen demnach eine hohe Aktivität und unter den Reaktionsbedingungen eine hohe mechanische Stabilität aufweisen.

Demgemäß wurde ein Verfahren zur Herstellung von Aminen durch Umsetzung von Aldehyden oder Ketonen bei erhöhter Temperatur und erhöhtem Druck mit Stickstoffverbindungen, ausgewählt aus der Gruppe Ammoniak, primäre und sekundäre Amine, und mit Wasserstoff in Gegenwart eines Katalysators enthaltend Kupfer gefunden, welches dadurch gekennzeichnet ist, dass die katalytisch aktive Masse des Katalysators vor der Reduktion mit Wasserstoff
20 bis 85 Gew.-% sauerstoffhaltige Verbindungen des Zirkoniums, berechnet als ZrO₂,
1 bis 30 Gew.-% sauerstoffhaltige Verbindungen des Kupfers, berechnet als CuO,
14 bis 70 Gew.-% sauerstoffhaltige Verbindungen des Nickels, berechnet als NiO,
0 bis 5 Gew.-% sauerstoffhaltige Verbindungen des Molybdäns, berechnet als MoO₃, und 0 bis 10 Gew.-% sauerstoffhaltige Verbindungen des Aluminiums, berechnet als Al₂O₃, enthält.

Im allgemeinen werden im erfindungsgemäßen Verfahren die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.

In diesem Zusammenhang werden die als Trägermaterialien verwendeten sauerstoffhaltigen Verbindungen des Zirkoniums und des Aluminiums als zur katalytisch aktiven Masse gehörig gewertet.

Die Katalysatoren werden dergestalt eingesetzt, dass man die katalytisch aktive, zu Pulver vermahlene Masse in das Reaktionsgefäss einbringt oder, dass man die katalytisch aktive Masse nach Mahlung, Vermischung mit Formhilfsmitteln, Formung und Temperung als Katalysatorformkörper - beispielsweise als Tabletten, Kugeln, Ringe, Extrudate (z.B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und der obengenannten Katalysatorträgermaterialien definiert und enthält im wesentlichen die Bestandteile sauerstoffhaltige Verbindungen des Zirkoniums, sauerstoffhaltige Verbindungen des Kupfers, sauerstoffhaltige Verbindungen des Nickels sowie optional sauerstoffhaltige Verbindungen des Molybdäns und/oder sauerstoffhaltige Verbindungen des Aluminiums.

Die Summe der obengenannten Bestandteile der katalytisch aktiven Masse, berechnet als ZrO₂, CuO, NiO, MoO₃ und Al₂O₃, beträgt üblicherweise 70 bis 100 Gew.-%, bevorzugt 80 bis 100 Gew.-%, besonders bevorzugt 90 bis 100 Gew.-%, insbesondere 95 bis 100 Gew.-%, ganz besonders bevorzugt 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten.

### Beispiele für solche Elemente bzw. deren Verbindungen sind:

Übergangsmetalle, wie Co bzw. CoO, Mn bzw. Manganoxide, Re bzw. Rheniumoxide, Cr bzw. Chromoxide, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. CeO₂ oder Pr bzw. Pr₂O₃; Alkalimetalloxide, wie Na₂O; Alkalimetallcarbonate; Erdalkalimetalloxide, wie SrO; Erdalkalimetallcarbonate, wie MgCO₃, CaCO₃ und BaCO₃; Boroxid (B₂O₃).

Die katalytisch aktive Masse der im erfindungsgemäßen Verfahren eingesetzten Katalysatoren enthält nach deren letzter Wärmebehandlung und vor der Reduktion mit Wasserstoff
20 bis 85 Gew.-%, bevorzugt 20 bis 84,9 Gew.-%, besonders bevorzugt 22 bis 65 Gew.-%, ganz besonders bevorzugt 25 bis 49,7 Gew.-%, sauerstoffhaltige Verbindungen des Zirkoniums, berechnet als ZrO₂,
1 bis 30 Gew.-%, besonders bevorzugt 5 bis 25 Gew.-%, ganz besonders bevorzugt 10 bis 25 Gew.-%, sauerstoffhaltige Verbindungen des Kupfers, berechnet als CuO,
14 bis 70 Gew.-%, besonders bevorzugt 29,7 bis 70 Gew.-%, ganz besonders bevorzugt 40 bis 60 Gew.-%, sauerstoffhaltige Verbindungen des Nickels, berechnet als NiO,
0 bis 5 Gew.-%, bevorzugt 0,1 bis 5 Gew.-%, besonders bevorzugt
0,3 bis 3,5 Gew.-%, sauerstoffhaltige Verbindungen des Molybdäns, berechnet als MoO₃, und
0 bis 10 Gew.-%, ganz besonders bevorzugt 0 bis 5 Gew.-%, sauerstoffhaltige Verbindungen des Aluminiums, berechnet als Al₂O₃.

Der Gehalt der katalytisch aktiven Masse an sauerstoffhaltigen Verbindungen des Aluminiums, berechnet als Al₂O₃, kann bis zu 10 Gew.-% betragen, wobei das Gewichtsverhältnis der sauerstoffhaltigen Verbindungen des Zirkoniums, berechnet als ZrO₂, zu den sauerstoffhaltigen Verbindungen des Aluminiums, berechnet als Al₂O₃, mindestens 2,2, bevorzugt mindestens 2,5, besonders bevorzugt mindestens 5, beträgt.

Bevorzugt werden im erfindungsgemäßen Verfahren Katalysatoren eingesetzt, deren katalytisch aktive Masse nach der letzten Wärmebehandlung und vor der Reduktion mit Wasserstoff weniger als 20 Gew.-%, bevorzugt weniger als 10 Gew.-%, insbesondere weniger als 5 Gew.-%, ganz besonders weniger als 1 Gew.-%, Kobalt, berechnet als CoO, enthält. Ganz besonders bevorzugt enthält die katalytisch aktive Masse keine katalytisch aktiven Mengen an Kobalt oder dessen Verbindungen.

Weiterhin beträgt bei den im erfindungsgemäßen Verfahren bevorzugt eingesetzten Katalysatoren das Molverhältnis von Nickel, berechnet als NiO, zu Kupfer, berechnet als CuO, größer 1, bevorzugt größer 1,2, besonders bevorzugt 1,8 bis 8,5.

Zur Herstellung der Katalysatoren sind verschiedenerlei Verfahrensweisen möglich. Sie sind beispielsweise durch Peptisieren pulvriger Mischungen der Hydroxide, Carbonate, Oxide und/oder anderer Salze der Komponenten mit Wasser und nachfolgendes Extrudieren und Tempern der so erhaltenen Masse erhältlich.

Im allgemeinen werden zur Herstellung der erfindungsgemäßen Katalysatoren jedoch Fällungsmethoden angewandt. So können sie beispielsweise durch eine gemeinsame Fällung der Nickel- und Kupferkomponenten aus einer diese Elemente enthaltenden, wässrigen Salzlösung mittels Mineralbasen in Gegenwart einer Aufschlämmung einer schwerlöslichen, sauerstoffhaltigen Zirkoniumverbindung und anschließendes Waschen, Trocknen und Calcinieren des erhaltenen Präzipitats erhalten werden. Als schwerlösliche, sauerstoffhaltige Zirkoniumverbindungen können beispielsweise Zirkoniumdioxid, Zirkoniumoxidhydrat, Zirkoniumphosphate, -borate und -silikate Verwendung finden. Die Aufschlämmungen der schwerlöslichen Zirkoniumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Zirkoniumverbindungen aus wässrigen Zirkoniumsalzlösungen mittels Mineralbasen erhalten.

Bevorzugt werden die erfindungsgemäßen Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Das Molybdän wird gegebenenfalls bevorzugt nachträglich eingebracht. Die Art der verwendeten Salze ist im allgemeinen nicht kritisch: Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit.
Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überläßt.

Die nach diesen Fällungsverfahren erhaltenen Niederschläge werden wie üblich zu den erfindungsgemäßen Katalysatoren weiterverarbeitet. Nach dem Waschen werden sie im allgemeinen bei 80 bis 200°C, vorzugsweise bei 100 bis 150°C, getrocknet und danach calciniert.
Die Calcinierung wird im allgemeinen bei Temperaturen zwischen 300 und 800°C, vorzugsweise bei 400 bis 600°C, insbesondere bei 450 bis 550°C ausgeführt.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Tablettenpresse zu Formlingen verpreßt und tempert. Die Tempertemperaturen entsprechen dabei im allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren werden als solche gelagert und ggf. gehandelt. Vor ihrem Einsatz als Katalysatoren zur hydrierenden Aminierung von Aldehyden oder Ketonen werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden. Zur Vorreduktion werden die Katalysatoren im allgemeinen zunächst bei 150 bis 200°C über einen Zeitraum von 12 bis 20 Stunden einer Stickstoff-Wasserstoff-Atmosphäre ausgesetzt und anschließend noch bis zu ca. 24 Stunden bei 200 bis 400°C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Ein besonderer Vorteil der im erfindungsgemäßen Verfahren eingesetzten Katalysatoren ist deren mechanische Stabilität, d.h. deren Härte. Die mechanische Stabilität wird durch die sogenannte Seitendruckfestigkeit bestimmt. Hierzu wird der Katalysatorformkörper, z. B. die Katalysatortablette, zwischen zwei parallelen Platten mit zunehmender Kraft belastet, wobei diese Belastung z.B. auf der Mantelseite von Katalysatortabletten erfolgen kann, bis ein Bruch des Katalysatorformkörpers eintritt. Die beim Bruch des Katalysatorformkörpers registrierte Kraft ist die Seitendruckfestigkeit.

### Amine der allgemeinen Formel I

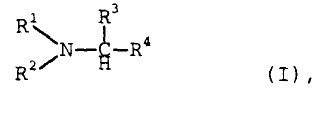

in der
- R¹, R²: Wasserstoff, C₁₋₂₀-Alkyl, C₃₋₁₂-Cycloalkyl, Aryl, C₇₋₂₀-Aralkyl und C₇₋₂₀-Alkylaryl oder gemeinsam (CH₂)ⱼ-X-(CH₂)ₖ,
- R₃, R₄: Wasserstoff, Alkyl, wie C₁₋₂₀₀-Alkyl, Cycloalkyl, wie C₃₋₁₂-Cycloalkyl, Hydroxyalkyl, wie C₁₋₂₀-Hydroxyalkyl, Aminoalkyl, wie C₁₋₂₀-Aminoalkyl, Alkanolaminoalkyl, wie C₁₋₂₀-Alkanolaminoalkyl, Alkoxyalkyl, wie C₂₋₃₀-Alkoxyalkyl, Dialkylaminoalkyl, wie C₃₋₃₀-Dialkylaminoalkyl, Alkylaminoalkyl, wie C₂₋₃₀-Alkylaminoalkyl, R⁵-(OCR⁶R⁷CR⁸R⁹)ₙ-(OCR⁶R⁷), Aryl, Heteroaryl, Aralkyl, wie C₇₋₂₀-Aralkyl, Heteroarylalkyl, wie C₄₋₂₀-Heteroarylalkyl, Alkylaryl, wie C₇₋₂₀-Alkylaryl, Alkylheteroaryl, wie C₄₋₂₀-Alkylheteroaryl und Y-(CH₂)ₘ-NR⁵-(CH₂)_{q} oder gemeinsam (CH₂)ₗ-X-(CH₂)ₘ oder
- R² und R⁴: gemeinsam (CH₂)ₗ-X-(CH₂)ₘ,
- R⁵, R¹⁰: Wasserstoff, C₁₋₄-Alkyl, C₇₋₂₀-Alkylphenyl,
- R⁶, R⁷, R⁸, R⁹: Wasserstoff, Methyl oder Ethyl,
- X: CH₂, CHR⁵, Sauerstoff (O) oder NR⁵,
- Y: N(R¹⁰)₂, Hydroxy, C₂₋₂₀-Alkylaminoalkyl oder C₃₋₂₀-Dialkylaminoalkyl,
- n: eine ganze Zahl von 1 bis 30 und
- j, k, l, m, q: eine ganze Zahl von 1 bis 4,
bedeuten, sind wirtschaftlich von besonderem Interesse.

Das erfindungsgemäße Verfahren findet daher bevorzugt zur Herstellung der Amine I Anwendung, indem man Aldehyde oder Ketone der Formel II bzw. III 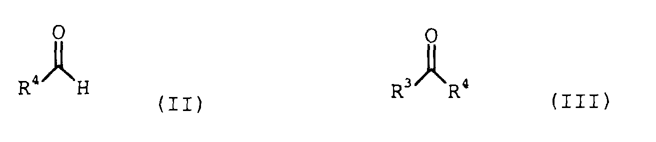 mit Stickstoffverbindungen der allgemeinen Formel IV 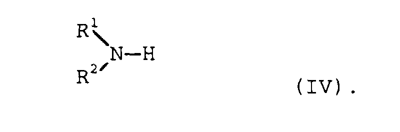 wobei R¹, R², R³ und R⁴ die oben genannten Bedeutungen haben, umgesetzt.

Wie aus den Definitionen für die Reste R² und R⁴ hervorgeht, kann die Umsetzung auch intramolekular in einem entsprechenden Aminoketon oder Aminoaldehyd erfolgen.

Die Substituenten R¹, R², R³, R⁴, R⁵, R⁶, R⁷, R⁸, R⁹, R¹⁰, die Variablen X, Y, und die Indizes j, k, l, m, n und q in den Verbindungen I, II, III und IV haben unabhängig voneinander folgende Bedeutungen:

### R¹, R², R³, R⁴, R⁵, R⁶, R⁷, R⁸, R⁹, R¹⁰

- Wasserstoff (H),

### R³, R⁴

- C₁₋₂₀₀-Alkyl, bevorzugt C₁₋₁₄-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl, besonders bevorzugt iso-Propyl, 2-Ethylhexyl, n-Decyl, 2-n-Propyl-n-heptyl, n-Tridecyl, 2-n-Butyl-n-nonyl und 3-n-Butyl-n-nonyl sowie bevorzugt C₄₀₋₂₀₀-Alkyl, wie Polybutyl, Polyisobutyl, Polypropyl, Polyisopropyl und Polyethyl, besonders bevorzugt Polybutyl und Polyisobutyl,
- C₁₋₂₀-Hydroxyalkyl, bevorzugt C₁₋₈-Hydroxyalkyl, besonders bevorzugt C₁₋₄-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-Hydroxy-methyl-ethyl,
- C₁₋₂₀-Aminoalkyl, bevorzugt C₁₋₈-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(Aminoethyl)aminoethyl und N-(Aminoethyl) aminomethyl,
- C₁₋₂₀-Hydroxyalkylaminoalkyl, bevorzugt C₁₋₈-Hydroxyalkylaminoalkyl, wie (2-Hydroxyethylamino)methyl, 2-(2-Hydroxyethylamino)ethyl und 3-(2-Hydroxyethylamino)propyl,
- C₂₋₃₀-Alkoxyalkyl, bevorzugt C₂₋₂₀-Alkoxyalkyl, besonders bevorzugt C₂₋₈-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl, besonders bevorzugt C₂- bis C₄-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl,
- R⁵-(OCR⁶R⁷CR⁸R⁹)ₙ-(OCR⁶R⁷), bevorzugt R⁵-(OCHR⁷CHR⁹)ₙ-(OCR⁶R⁷), besonders bevorzugt R⁵-(OCH₂CHR⁹)ₙ-(OCR⁶R⁷),
- C₃₋₃₀-Dialkylaminoalkyl, bevorzugt C₃₋₂₀-Dialkylaminoalkyl, besonders bevorzugt C₃₋₁₀- N,N-Dialkylaminoalkyl, wie N,N-Dimethylaminomethyl, 2-(N,N-Dibutylamino)methyl, 2-(N,N-Dimethylamino)ethyl, 2-(N,N-Diethylamino)ethyl, 2-(N,N-Dibutylamino)ethyl, 2-(N,N-Di-n-propylamino)ethyl und 2-(N,N-Di-isopropylamino)ethyl, (R⁵)₂N-(CH₂)_{q},
- C₂₋₃₀-Alkylaminoalkyl-, bevorzugt C₂₋₂₀- Alkylaminoalkyl, besonders bevorzugt C₂₋₈- Alkylaminoalkyl, wie Methylaminomethyl, Methylaminoethyl, Ethylaminomethyl, Ethylaminoethyl und iso-Propylaminoethyl, (R⁵) HN-(CH₂)_{q},
- Y-(CH₂)ₘ-NR⁵-(CH₂)_{q},
- C₄₋₂₀-Heteroarylalkyl, wie Pyrid-2-yl-methyl, Furan-2-yl-methyl, Pyrrol-3-yl-methyl und Imidazol-2-yl-methyl,
- C₄₋₂₀-Alkylheteroaryl, wie 2-Methyl-3-pyridinyl, 4,5-Dimethyl-imidazol-2-yl, 3-Methyl-2-furanyl und 5-Methyl-2-pyrazinyl,
- Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,

### R¹, R², R³, R⁴

- C₃₋₁₂-Cycloalkyl, bevorzugt C₃₋₈-Cycloalkyl, wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇₋₂₀-Alkylaryl, bevorzugt C₇₋₁₂-Alkylphenyl, wie 2-Methylphenyl, 3-Methylphenyl, 4-Methylphenyl, 2,4-Dimethylphenyi, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
- C₇₋₂₀-Aralkyl, bevorzugt C₇₋₁₂-Phenylalkyl, wie Benzyl, p-Methoxybenzyl, 3,4-Dimethoxybenzyl, 1-Phenethyl, 2-Phenethyl, 1-Phenyl-propyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- R³ und R⁴ oder R² und R⁴ gemeinsam eine -(CH₂)ₗ-X-(CH₂)ₘ- Gruppe, , wie -(CH₂)₃-, -(CH₂)₄-, -(CH₂)₅-, -(CH₂)₆-, -(CH₂)₇-, -(CH₂)-O-(CH₂)₂-, -(CH₂)-NR⁵-(CH₂)₂-, -(CH₂)₂-O-(CH₂)₂-, -(CH₂)₂-NR⁵-(CH₂)₂-, -CH₂-O-(CH₂)₃-, -CH₂-NR⁵-(CH₂)₃-,

### R¹, R²

- C₁₋₂₀-Alkyl, bevorzugt C₁₋₈-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁₋₄-Alkyl wie Methyl, Ethyl, n-Propyl, iso-Propyl, n-Butyl, iso-Butyl, sec-Butyl und tert.-Butyl,
- R¹ und R² gemeinsam eine -(CH₂)ⱼ-X-(CH₂)ₖ- Gruppe, wie -(CH₂)₃-, -(CH₂)₄-, -(CH₂)₅-, -(CH₂)₆-, -(CH₂)₇-, -(CH₂)-O-(CH₂)₂-, -(CH₂)-NR⁵-(CH₂)₂-, -(CH₂)₂-O-(CH₂)₂-, -(CH₂)₂-NR⁵-(CH₂)₂-, -CH₂-O-(CH₂)₃-, -CH₂-NR⁵-(CH₂)₃-,

### R⁵, R¹⁰

- C₁₋₄-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
- C₇₋₄₀-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl,

### R⁶, R⁷, R⁸, R⁹

- Methyl und Ethyl, bevorzugt Methyl,

### X

- CH₂, CHR⁵, Sauerstoff oder NR⁵,

### Y

- N(R¹⁰)₂,
- Hydroxy,
- C₂₋₂₀-Alkylaminoalkyl, bevorzugt C₂₋₁₆-Alkylaminoalkyl, wie Methylaminomethyl, Methylaminoethyl, Ethylaminomethyl, Ethylaminoethyl und iso-Propylaminoethyl,
- C₃₋₂₀-Dialkylaminoalkyl, bevorzugt C₃₋₁₆-Dialkylaminoalkyl, wie Dimethylaminomethyl, Dimethylaminoethyl, Diethylaminoethyl, Di-n-propylaminoethyl und Di-iso-propylaminoethyl,

### j, l

- eine ganze Zahl von 1 bis 4 wie 1, 2, 3 und 4, bevorzugt 2 und 3, besonders bevorzugt 2,

### k, m, q

- eine ganze Zahl von 1 bis 4 wie 1, 2, 3 und 4, bevorzugt 2, 3 und 4, besonders bevorzugt 2 und 3,

### n

- eine ganze Zahl von 1 bis 10, bevorzugt eine ganze Zahl von 1 bis 8 wie 1, 2, 3, 4, 5, 6, 7 oder 8, besonders bevorzugt eine ganze Zahl von 1 bis 6 wie 1, 2, 3, 4, 5 oder 6.

Als im erfindungsgemäßen Verfahren einsetzbare Ketone eignen sich praktisch alle aliphatischen und aromatischen Ketone. Die aliphatischen Ketone können geradkettig, verzweigt oder zyklisch sein, die Ketone können Heteroatome enthalten. Hinsichtlich der Kohlenstoffzahl der aminierbaren Ketone sind bislang keine Beschränkungen bekannt. Die Ketone können ferner Substituenten tragen, welche sich unter den Bedingungen der hydrierenden Aminierung inert verhalten, beispielsweise Alkoxy-, Alkenyloxy-, Alkylamino- oder Dialkylaminogruppen. Sollen mehrwertige Ketone aminiert werden, so hat man es über die Steuerung der Reaktionsbedingungen in der Hand, Aminoalkohole, zyklische Amine oder mehrfach aminierte Produkte zu erhalten.

### Bevorzugt werden beispielsweise die folgenden Ketone aminierend hydriert:

Aceton, Ethylmethylketon, Methylvinylketon, Isobutylmethylketon, 3-Methylbutan-2-on, Diethylketon, Tetralon, Acetophenon, p-Methyl-acetophenon, p-Methoxy-acetophenon, m-Methoxy-acetophenon, 1-Acetyl-naphthalin, 2-Acetyl-naphthalin, 1-Phenyl-3-butanon, Cyclobutanon, Cyclopentanon, Cyclopentenon, Cyclohexanon, Cyclohexenon, 2,6-Dimethylcyclohexanon, Cycloheptanon, Cyclododecanon, Acetylaceton, Methylglyoxal und Benzophenon.

Als im erfindungsgemäßen Verfahren einsetzbare Aldehyde eignen sich praktisch alle aliphatischen und aromatischen Aldehyde. Die aliphatischen Aldehyde können geradkettig, verzweigt oder zyklisch sein, die Aldehyde können Heteroatome enthalten. Hinsichtlich der Kohlenstoffzahl der aminierbaren Aldehyde sind bislang keine Beschränkungen bekannt. Die Aldehyde können ferner Substituenten tragen, welche sich unter den Bedingungen der hydrierenden Aminierung inert verhalten, beispielsweise Alkoxy-, Alkenyloxy-, Alkylamino- oder Dialkylaminogruppen. Sollen mehrwertige Aldehyde oder Ketoaldehyde aminiert werden, so hat man es über die Steuerung der Reaktionsbedingungen in der Hand, Aminoalkohole, cyclische Amine oder mehrfach aminierte Produkte zu erhalten.

### Bevorzugt werden beispielsweise die folgenden Aldehyde aminierend hydriert:

Formaldehyd, Acetaldehyd, Propionaldehyd, n-Butyraldehyd, Isobutyraldehyd, Pivalinaldehyd, n-Pentanal, n-Hexanal, 2-Ethylhexanal, 2-Methylpentanal, 3-Methylpentanal, 4-Methylpentanal, Glyoxal, Benzaldehyd, p-Methoxybenzaldehyd, p-Methylbenzaldehyd, Phenylacetaldehyd, (p-Methoxy-phenyl) acetaldehyd, (3,4-Dimethoxy-phenyl) acetaldehyd, 4-Formyltetrahydropyran, 3- Formyltetrahydrofuran, 5-Formylvaleronitril, Citronellal, Acrolein, Methacrolein, Ethylacrolein, Citral, Crotonaldehyd, 3-Methoxypropionaldehyd, 3-Aminopropionaldehyd, Hydroxypivalinaldehyd, Dimethylolpropionaldehyd, Dimethylolbutyraldehyd, Furfural, Glyoxal sowie hydroformylierte Oligomere und Polymere, wie z. B. hydroformyliertes Polyisobuten (Polyisobutenaldehyd, PIBA) oder durch Metathese von 1-Penten und Cyclopenten erhaltenes und hydroformyliertes Oligomer.

Als Aminierungsmittel bei der hydrierenden Aminierung von Aldehyden und Ketonen können sowohl Ammoniak als auch primäre oder sekundäre, aliphatische oder cycloaliphatische Amine eingesetzt werden.

Bei Verwendung von Ammoniak als Aminierungsmittel werden die Carbonylgruppen zunächst in freie Aminogruppen (-NH₂) umgewandelt. Die so gebildeten primären Amine können mit Hydroxyl- oder weiteren Carbonylgruppen zu den entsprechenden sekundären Aminen und diese wiederum mit Hydroxyl- oder weiteren Carbonylgruppen zu den entsprechenden, symmetrischen tertiären Aminen reagieren. Je nach Zusammensetzung des Reaktionsansatzes und je nach den angewandten Reaktionsbedingungen - Druck, Temperatur, Reaktionszeit - lassen sich auf diese Weise je nach Wunsch bevorzugt primäre, sekundäre oder tertiäre Amine darstellen.

Aus mehrwertigen Aldehyden oder Ketonen lassen sich auf diese Weise durch intramolekulare hydrierende Aminierung zyklische Amine wie Pyrrolidine, Piperidine, Hexamethylenimine, Piperazine und Morpholine herstellen.

Ebenso wie Ammoniak lassen sich primäre oder sekundäre Amine als Aminierungsmittel verwenden.

Bevorzugt werden diese Aminierungsmittel zur Herstellung unsymmetrisch substituierter Di- oder Trialkylamine, wie Ethyldiisopropylamin und Ethyldicyclohexylamin verwendet. Bevorzugt werden beispielsweise die folgenden Mono- und Dialkylamine als Aminierungsmittel verwendet: Methylamin, Dimethylamin, Ethylamin, Diethylamin, Propylamin, Diisopropylamin, Butylamin, Pentylamin, Hexylamin und Cyclohexylamin.

Das Aminierungsmittel kann bezüglich der aminierend zu hydrierenden Carbonylgruppe in stöchiometrischer Menge eingesetzt werden.
Bevorzugt wird jedoch mit einem Überschuß an Aminierungsmittel gearbeitet und zwar im allgemeinen mit einem mehr als 5molaren Überschuß pro Mol aminierend zu hydrierender Carbonylgruppe. Speziell Ammoniak wird im allgemeinen mit einem 5 bis 250-fachen, bevorzugt 10 bis 100-fachen, insbesondere 25 bis 80-fachen molaren Überschuß pro Mol umzusetzender Carbonylgruppen eingesetzt.
Höhere Überschüsse sowohl an Ammoniak als auch an primären oder sekundären Aminen sind möglich.

Das erfindungsgemäße Verfahren läßt sich diskontinuierlich oder bevorzugt kontinuierlich wie folgt durchführen, wobei der Katalysator bevorzugt als Festbett im Reaktor angeordnet ist.

Die Aminierung der Aldehydgruppen oder Ketogruppen des Edukts kann in der Flüssigphase oder in der Gasphase durchgeführt werden.

Üblicherweise arbeitet man bei der Umsetzung bei Temperaturen von 80 bis 250°C, bevorzugt 80 bis 230°C, besonders bevorzugt 80 bis 200°C, ganz besonders bevorzugt 80 bis 155°C.

Im allgemeinen wird die Reaktion bei einem Druck von 1 bis 400 bar (0,1 bis 40 MPa) ausgeführt. Bevorzugt werden Drücke von 10 bis 250 bar, insbesondere von 20 bis 200 bar, angewandt.

Die Anwendung höherer Temperaturen und eines höheren Gesamtdrukkes ist möglich. Der Gesamtdruck im Reaktionsgefäß, welches sich aus der Summe der Partialdrücke des Aminierungsmittels, der Carbonylkomponente, der gebildeten Reaktionsprodukte sowie des ggf. mitverwendeten Lösungsmittels bei den angegebenen Temperaturen ergibt, wird zweckmäßigerweise durch Aufpressen von Wasserstoff auf den gewünschten Reaktionsdruck eingestellt.

Der Wasserstoff wird der Reaktion im allgemeinen in einer Menge von 5 bis 400 N1, bevorzugt in einer Menge von 50 bis 200 N1 pro Mol Carbonylkomponente zugeführt, wobei die Literangaben jeweils auf Normalbedingungen umgerechnet wurden (S.T.P.).

Die Umsetzung erfolgt im allgemeinen ohne zusätzliches Lösungsmittel. Bei der Umsetzung hochmolekularer hochviskoser oder bei Raumtemperatur fester Ausgangsverbindungen oder Produkte kann es vorteilhaft sein ein unter den Reaktionsbedingungen inertes Lösungsmittel, wie Methanol, Ethanol, Propanol, Tetrahydrofuran, Dioxan, N-Methylpyrrolidon, Mihagol oder Ethylenglykoldimethylether mitzuverwenden.

Es kann für die Selektivität des vorliegenden Verfahrens vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Praktisch geht man bei der Durchführung im allgemeinen so vor, dass man dem Katalysator, der sich üblicherweise in einem bevorzugt von außen beheizten Festbettreaktor befindet, bei der gewünschten Reaktionstemperatur und dem gewünschten Druck den Aldehyd oder das Keton und das Aminierungsmittel simultan zuführt. Dabei belastet man den Katalysator im allgemeinen mit 0,02 bis 3, bevorzugt 0,05 bis 2, und besonders bevorzugt mit 0,1 bis 1,6 l Aldehyd oder Keton pro Liter Katalysator und Stunde. Hierbei ist es zweckmäßig, die Reaktanten bereits vor der Zuführung in das Reaktionsgefäß zu erwärmen und zwar bevorzugt auf die Reaktionstemperatur.

Der Reaktor kann sowohl in der Sumpf- als auch in der Rieselfahrweise betrieben werden, d. h. man kann die Reaktanten sowohl von unten nach oben als auch von oben nach unten durch den Reaktor leiten. Es versteht sich von selbst, dass sich das Verfahren sowohl diskontinuierlich als auch kontinuierlich durchführen läßt.
In beiden Fällen kann das überschüssige Aminierungsmittel zusammen mit dem Wasserstoff im Kreis geführt werden. Ist der Umsatz bei der Reaktion nicht vollständig, so kann das nicht umgesetzte Ausgangsmaterial ebenfalls in die Reaktionszone zurückgeführt werden.

Aus dem Reaktionsaustrag werden nachdem dieser zweckmäßigerweise entspannt worden ist, das überschüssige Aminierungsmittel und der Wasserstoff entfernt und die erhaltenen aminierten Produkte durch Destillation, Flüssigextraktion oder Kristallisation gereinigt.

Das überschüssige Aminierungsmittel und der Wasserstoff werden vorteilhaft wieder in die Reaktionszone zurückgeführt. Das gleiche gilt für die eventuell nicht oder nicht vollständig umgesetzte Carbonylkomponente oder durch Hydrierung entstandener Alkohol.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der destillativen Aufarbeitung des Reaktionsproduktes aus diesem entfernt.

Die durch das erfindungsgemäße Verfahren erhältlichen Amine eignen sich u. a. als Zwischenprodukte bei der Herstellung von Kraftstoffadditiven (US-A-3 275 554; DE-A-21 25 039 und DE-A-36 11 230), Tensiden, Arznei- und Pflanzenschutzmitteln sowie von Vulkanisationsbeschleunigern.

### Beispiele

### Herstellung von Katalysator A

Eine wässrige Lösung aus Nickelnitrat, Kupfernitrat und Zirkoniumacetat, die 4,48 % NiO, 1,52 % CuO und 2,82 % ZrO₂ enthielt, wurde gleichzeitig in einem Rührgefäß in einem konstanten Strom mit einer 20 %igen wässrigen Natriumcarbonatlösung bei einer Temperatur von 70°C, so gefällt, dass der mit einer Glaselektrode gemessene pH-Wert von 7,0 aufrechterhalten wurde.

Die erhaltene Suspension wurde filtriert und der Filterkuchen mit vollentsalztem Wasser gewaschen bis die elektrische Leitfähigkeit des Filtrats ca. 20 mS betrug. Anschließend wurden 1,5 % MoO₃ als wässrige Lösung von Ammoniumheptamolybdat untergerührt. Danach wurde der Filterkuchen bei einer Temperatur von 150°C in einem Trockenschrank oder einem Sprühtrockner getrocknet. Das auf diese Weise erhaltene Hydroxidcarbonatgemisch wurde nun bei einer Temperatur von 500°C über einen Zeitraum von 4 Stunden getempert.

### Der so erhaltene Katalysator hatte die Zusammensetzung:

51 Gew.-% NiO, 17 Gew.-% CuO, und 30,5 Gew.-% ZrO₂ und 1,5 Gew.-% MoO₃. Das Katalysatorpulver wurde mit 3 Gew.-% Graphit vermischt und zu 6 x 3 mm-Tabletten verformt.

### Ermittlung der mechanische Stabilität von Katalysator A unter Reaktionsbedingungen (Kochtest):

Ein Autoklav wurde mit 30 g Katalysator A (im Korb), 67 ml Morpholin, 67 ml Diethylenglykol und 16 ml Wasser befüllt. Nach Verschließen des Autoklaven wurde der Autoklav mit Argon gespült.
Der Autoklaveninhalt wurde gerührt (700 U/min). Anschließend wurden 50 bar H₂ aufgepreßt und der Reaktorinhalt innerhalb von 120 Min. auf die gewünschte Temperatur, z. B. 150°C oder 200°C, aufgeheizt. Der Druck wurde mit H₂ bis auf 200 bar erhöht und 16 h bei der jeweiligen Temperatur gerührt. Nach Abkühlung wurde sehr langsam entspannt (während ca. 15 Min.), so dass in den Katalysatorporen befindliche Reaktionsteilnehmer nicht plötzlich verdampfen und die Katalysatortablette nicht sprengen. Die Tabletten wurden anschließend auf ihre Härte überprüft.
Wie aus der folgenden Ergebnistabelle hervorgeht, weist der Katalysator A bei deutlich niedrigeren Temperaturen als 200°C, z.B. bei Temperaturen von 80 bis 155°C, nach Versuchsende überraschenderweise eine wesentlich höhere Seitendruckfestigkeit auf als nach Versuchsende eines Versuchs bei 200°C.
Dies Ergebnis steht im Einklang mit den Ergebnissen zur hydrierenden Aminierung von Anisaldehyd (siehe Beispiel 1).

| Seitendruckfestigkeit des Katalysators A (in Newton [N]) | | |
|---|---|---|
| in reduzierter Form vor dem Kochtest: | nach dem Kochtest bei 200 bar und 200°C: | nach dem Kochtest bei 200 bar und 150°C: |
| 131 | 6 | 30 |

### Beispiel 1:

### Allgemeine Arbeitsvorschrift zur kontinuierlichen Aminierung von Carbonylverbindungen:

### Ein kontinuierlich betriebener Druckreaktor wurde mit 500 cm³ Katalysator A gefüllt und stündlich mit Carbonylverbindung und flüssigem Ammoniak beschickt. Die Katalysatoren wurden reduziert-passiviert eingebaut. Ohne Nachreduktion wurden die jeweiligen Reaktionsbedingungen eingestellt. Aus den Reaktionsausträgen wurde nach deren Entspannung überschüssiges Ammoniak abdestilliert. Die gesammelten Reaktionsausträge wurden jeweils gaschromatographisch analysiert (Die Einstellungen können der folgenden Tabelle entnommen werden):

### Kontinuierliche Aminierung von monomeren Verbindungen:

| **Carbonylverbindung** | **Amin** | **Temp.** | **Druck** | **Katalysatorbelastung** | **Amin/Carbonyl- Verbindung** | **Ausbeute******* | **Bemerkung** |
|---|---|---|---|---|---|---|---|
| | | [°c] | [bar] | [kg/l*h] | mol/mol | [%] | |
| | | | | | | Isopropylamin | |
| Aceton | NH₃ | 130 | 10 | 0,190 | 5,2 | 95,2 | 3,1 % Isopropanol, 1,2 % Diisopropanol |
| Aceton | NH₃ | 210 | 25 | 0,380 | 5,2 | 82,3 | 3,3 % Isopropanol, 13,5 % Di-isopropanol |
| Aceton | NH₃ | 210 | 20 | 0,644 | 6,6 | 87,7 | 3,1 % Isopropanol, 8,4 % Di-isopropanol |
| | | | | | | | |

| | | | | | | 4-Methoxybenzylamin | |
|---|---|---|---|---|---|---|---|
| Anisaldehyd | NH₃ | 120 ¹⁾ | 200 | 0,6 | 6,5 | 99,2 | 0,5 % Nebenprodukte |
| | | 130 ²⁾ | 200 | 0,6 | 6,5 | 98,6 | 0,7 % Nebenprodukte |
| | | 200 ³⁾ | 200 | 0,6 | 6,5 | 91,3 | 7,5 % Nebenprodukte |
| | | | | | | | |

| | | | | | | Cyclopentylamin | |
|---|---|---|---|---|---|---|---|
| Cyclopentanon | NH₃ | 150 | 200 | 0,76 | 13,5 | 92 | 6 % Cyclopentanol |
| Cyclopentanon | NH₃ | 160 | 200 | 0,76 | 13,5 | 94 | 4 % Cyclopentanol |
| Cyclopentanon | NH₃ | 170 | 200 | 0,76 | 13,5 | 97 | 1 % Cyclopentanol |
| | | | | | | | |

| | | | | | | Propylamine | |
|---|---|---|---|---|---|---|---|
| Propionaldehyd | NH₃ | 110 | 140 | 0,48 | 1,2 | 93 | 49 % MPA,40 % DPA, 4 % TPA, 2 % n-Propanol |
| Propionaldehyd | NH₃ | 150 | 140 | 0,48 | 1,2 | 97 | 53 % MPA, 39 % DPA, 5 % TPA, 1 % n-Propanol |
| | | | | | | | |

| | | | | | | 3-Pentylamin | |
|---|---|---|---|---|---|---|---|
| Diethylketon | NH₃ | 130 | 70 | 0,33 | 6,5 | 96 | 3 % 3-Pentanol |
| | | | | | | | |

| | | | | | | 2-Butylamin | |
|---|---|---|---|---|---|---|---|
| Methylethylketon | NH₃ | 160 | 200 | 0,48 | 10 | 95 | 2 % Sek. Butanol |
| Methylethylketon | NH₃ | 180 | 200 | 0,48 | 10 | 96 | 2 % Sek. Butanol |

| | | | | | | | |
|---|---|---|---|---|---|---|---|
| * Alle Reaktionen wurden bei Vollumsatz durchgeführt Anmerkungen zu den Umsetzungen von Anisaldehyd mit Ammoniak: | | | | | | | |
| 1) Die Seitendruckfestigkeit des nach Versuchsende ausgebauten Katalysators betrug 48 N. | | | | | | | |
| 2) Die Seitendruckfestigkeit des nach Versuchsende ausgebauten Katalysators betrug 37 N. | | | | | | | |
| 3) Die Seitendruckfestigkeit des nach Versuchsende ausgebauten Katalysators betrug 5 N. | | | | | | | |

### Beispiel 2:

### Kontinuierliche Aminierung von Polyisobutenaldehyd

### Ein kontinuierlich betriebener Hochdruckreaktor wurde mit 500 cm³ Katalysator A gefüllt und stündlich mit 500/750/1000 cm³ Polyisobuten-oxo-aldehyd (45 Gew.-%ige Lösung in Mihagol, CO-Zahl: 13 mg KOH/g, OH-Zahl: 17 mg KOH/g) und 280/425/565 cm³ flüssigem Ammoniak beschickt. Die Katalysatortemperatur wurde auf 190°C und der Druck im Reaktor, durch gleichzeitiges Aufpressen von Wasserstoff, auf 200 bar eingestellt. Aus den Reaktionsaustrag wurde nach dessen Entspannung überschüssiges Ammoniak abdestilliert. Die Analyse ergab folgende Werte:

### Aminierung von hydroformyliertem Polyisobuten (45 Gew.-% in Mihagol) bei 190°C/200 bar mit Ammoniak:

| Belastung [kg/l*h] | Amin/Carbonyl [mol/mol] | OH-Zahl [mg KOH/g] | CO-Zahl [mg KOH/g] | Aminzahl [mg KOH/g] | 2°+3° Aminzahl [mg KOH/g] | Acetyl.-Zahl [mg KOH/g] |
|---|---|---|---|---|---|---|
| Pib-Oxo | | 17 | 13 | | | |
| | | | | | | |
| 0,8 | 80 | <1 | <1 | 22 | 2 | 24 |
| 1,2 | 80 | <1 | <1 | 22 | 1 | 22 |
| 1,6 | 80 | <1 | <1 | 21 | 1 | 23 |

### Beispiel 3:

### Diskontinuierliche Aminierung hydroformyliertem Cyclopentenoligomer mit Ammoniak:

1. Der vorreduzierte und passivierte Katalysator A (15 ml) wurde nach Einbau in einem Katalysatorkorb und Dichtigkeitsprüfung des Autoklaven (300 ml Rührautoklav) unter 20 bar Wasserstoffdruck auf 200°C aufgeheizt, wobei sich ein Druck von ca. 30 bis 39 bar einstellte. Dann wurde auf 100 bar mit Wasserstoff ergänzt und der Katalysator aktiviert. Nach 16 h bei 200°C wurde abgekühlt und entspannt. Nach Aktivierung des Katalysators wurde der Druckbehälter evakuiert und 50 ml des umzusetzenden Edukts (hydroformyliertes, durch Metathese von 1-Penten und Cyclopenten erhaltenes Oligomer, Kennzahlen siehe folgende Tabelle) als 30%ige Lösung in Tetrahydrofuran in den Autoklaven unter Luftabschluß eingesogen und mit Stickstoff belüftet. Anschließend wurden 73 ml Ammoniak und 30 bar Wasserstoff aufgepreßt, auf die Endtemperatur (185°C) aufgeheizt, Wasserstoff auf den Enddruck nachgepreßt (200 bar) und für 20 h umgesetzt. Danach wurde abgekühlt, entspannt und der Autoklaveninhalt mit THF herausgelöst. Nach Abfiltrieren der festen Bestandteile und Abtrennen des THF am Rotationsverdampfer wurden 18,3 g eines flüssigen, farblosen Produkts erhalten.
2. 15 ml des Katalysators A wurden reduziert/passiviert eingebaut, direkt angefahren und an diesem Katalysator 60 ml Edukt (hydroformyliertes, durch Metathese von 1-Penten und Cyclopenten erhaltenes Oligomer, Kennzahlen siehe folgende Tabelle) als 50%ige Lösung in Tetrahydrofuran mit 50 ml Ammoniak analog bei 200°C und 220 bar für 20 h umgesetzt. Danach wurde abgekühlt, entspannt und der Autoklaveninhalt mit THF ausgebaut. Nach Abfiltrieren der festen Bestandteile und Abtrennen des THF am Rotationsverdampfer wurden 20,3 g eines flüssigen, farblosen Produkts erhalten; die Kennzahlen sind in der folgenden Tabelle wiedergegeben.
3. 150 ml des Katalysators A wurden in einem 2,5 l Rührautoklaven in einem Katalysatorkorb reduziert/passiviert eingebaut, direkt angefahren und an diesem Katalysator 600 ml Edukt (hydroformyliertes, durch Metathese von 1-Penten und Cyclopenten erhaltenes Oligomer, Kennzahlen s. Tabelle 3) als 50%ige Lösung in Tetrahydrofuran mit 500 ml Ammoniak analog bei 200°C und 220 bar für 48 h umgesetzt. Danach wurde abgekühlt, entspannt und der Autoklaveninhalt mit Toluol herausgelöst. Die festen Bestandteile wurden durch Filtration abgetrennt und das Lösungsmittel am Rotationsverdampfer entfernt, wobei 314 g einer farblosen Flüssigkeit erhalten wurden; die Kennzahlen sind in der folgenden Tabelle wiedergegeben.

### Diskontinuierliche Aminierung von hydroformyliertem Cyclopentenoligomer:

| Verhältnis Edukt:NH₃ | Temp. [°C] | Druck [bar] | Dauer [h] | OH-Zahl [mg KOH/g] | Co-Zahl [mg KOH/g] | Amin-Zahl [mg KOH/g] | Tert. Aminzahl [mg KOH/g] |
|---|---|---|---|---|---|---|---|
| 1:0 | | | | 27 | 307 | 0 | 0 |
| 1:25 | 185 | 200 | 20 | 33 | 3 | 252,7 | 7 |
| 1:10 | 200 | 220 | 20 | <1 | <1 | 231,5 | 9,9 |
| 1:10 | 200 | 220 | 48 | 15 | | 264 | 12,4 |

### Beispiel 4:

### Allgemeine Arbeitsvorschrift zur aminierenden Hydrierung in diskontinuierlicher Reaktionsführung:

In einem 1,2 l Autoklaven wurden 30 g Katalysator A vorgelegt und unter Stickstoffatmosphäre Ammoniak aufgepreßt. Nach Aufheizen auf 150°C (Druck beträgt ca. 170 bar) wurde innerhalb von 1 h die Carbonylverbindung zugepumpt (vorrübergehender Druckanstieg über 220 bar, bei hochsiedenden Ketonen wurde das Edukt zusammen mit dem Katalysator vorgelegt) und 1 h nachgerührt. Anschließend wurde in Anwesenheit von Wasserstoff bei einem Gesamtdruck bis zu 250 bar bis zur Druckkonstanz über 3 h hydriert (Dauer: 11 bis 15 h). Der Katalysator wurde jeweils in vorreduzierter passivierter Form als Pulver eingesetzt.
Die Zusammensetzung der Reaktionsausträge wurde gaschromatographisch ermittelt (Säule: 50 m OV 1701).

### Diskontinuierliche Aminierung von monomeren Verbindungen:

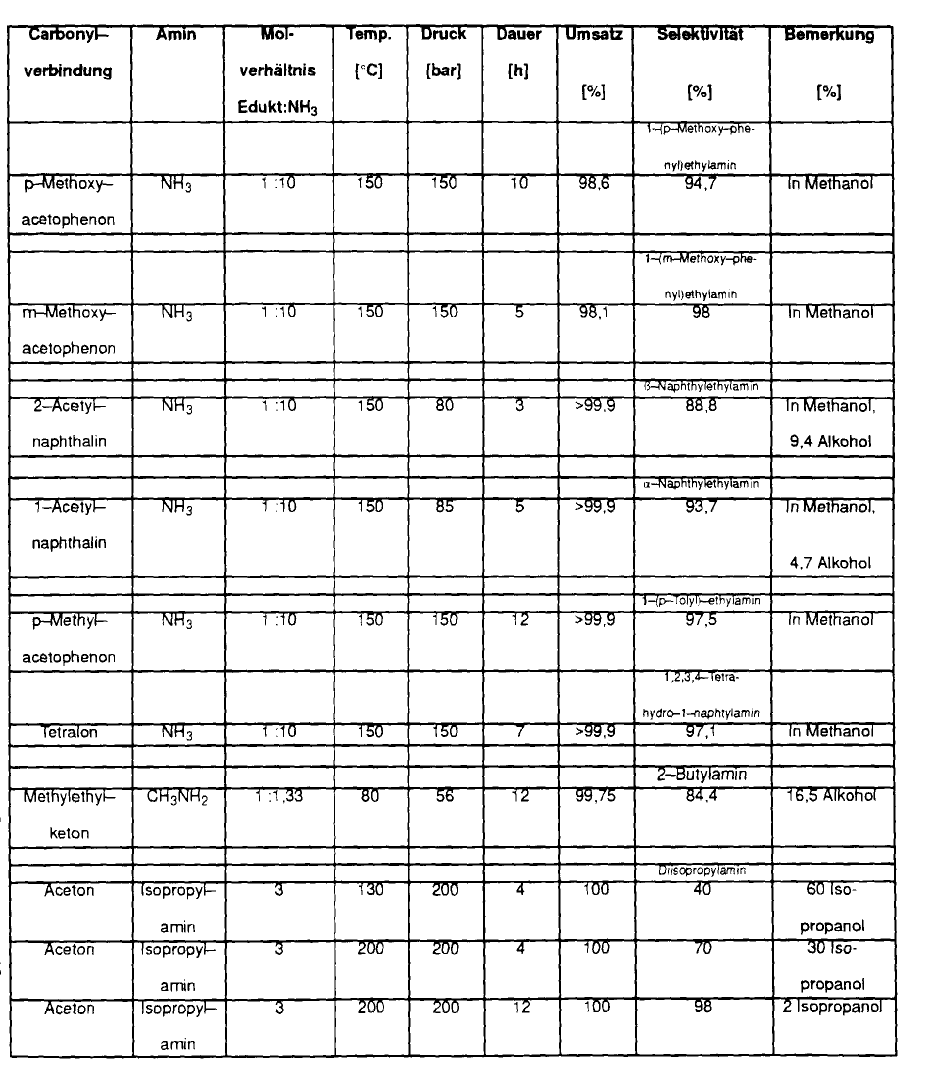

### Vergleichsbeispiele:

### Arbeitsvorschrift zur kontinuierlichen Aminierung von Aceton

Ein kontinuierlich betriebener Reaktor (Druck 10 bis 25 bar) wurde mit 100 cm³ Katalysator A gefüllt und mit Aceton und Ammoniak beschickt. Die Katalysatoren wurden reduziert-passiviert eingebaut. Ohne Nachreduktion wurden die jeweiligen Reaktionsbedingungen eingestellt und bei Vollumsatz gefahren. Aus den Reaktionsausträgen wurde nach deren Entspannung überschüssiges Ammoniak abdestilliert. Die gesammelten Reaktionsausträge wurden jeweils gaschromatographisch analysiert. Die jeweiligen Reaktionsbedingungen und Ergebnisse sind in der folgenden Tabelle zusammengefasst.
Zusammensetzung von Kat. B: 47 Gew.-% CuO, 10 Gew.-% NiO, 43 Gew.-% Al₂O₃
Zusammensetzung von Kat. C: 53 Gew.-% CuO, 47 Gew.-% TiO₂

Der Vergleich zeigt, daß der Katalysator A für die Herstellung von Monoisopropylamin die höchste Selektivität besitzt. Weiterhin ist bei Katalysator A der Anteil an unerwünschtem Alkohol (Isopropanol) am geringsten. Insbesondere Katalysator C zeigt eine starke Tendenz zur Bildung von sekundären Aminen.

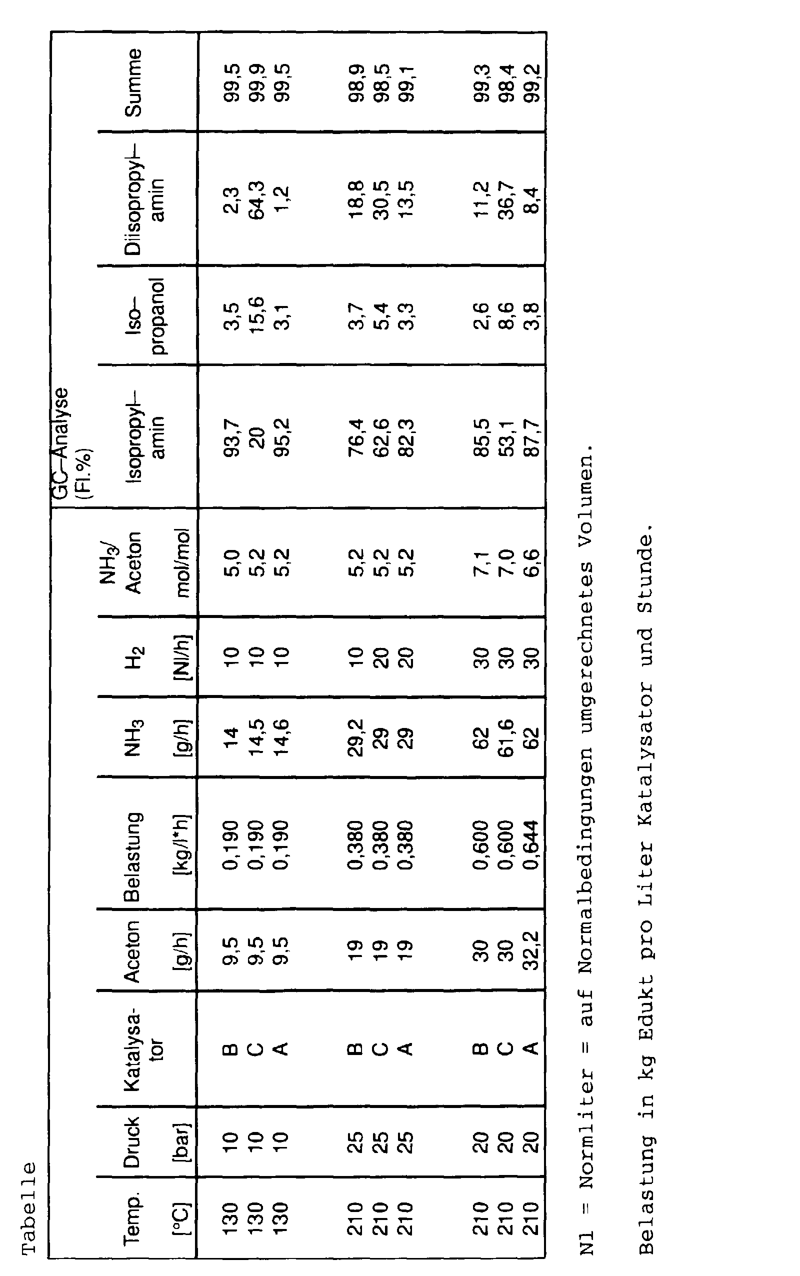

## Patentansprüche

1. Verfahren zur Herstellung von Aminen durch Umsetzung von Aldehyden oder Ketonen bei erhöhter Temperatur und erhöhtem Druck mit Stickstoffverbindungen, ausgewählt aus der Gruppe Ammoniak, primäre und sekundäre Amine, und mit Wasserstoff in Gegenwart eines Katalysators enthaltend Kupfer, dadurch gekennzeichnet, dass die katalytisch aktive Masse des Katalysators vor der Reduktion mit Wasserstoff
20 bis 85 Gew.-% sauerstoffhaltige Verbindungen des Zirkoniums, berechnet als ZrO₂,
1 bis 30 Gew.-% sauerstoffhaltige Verbindungen des Kupfers, berechnet als CuO,
14 bis 70 Gew.-% sauerstoffhaltige Verbindungen des Nickels, berechnet als NiO,
0 bis 5 Gew.-% sauerstoffhaltige Verbindungen des Molybdäns, berechnet als MoO₃,
und 0 bis 10 Gew.-% sauerstoffhaltige Verbindungen des Aluminiums, berechnet als Al₂O₃, enthält.

2. Verfahren nach Anspruch 1, dadurch gekennzeichnet, dass die katalytisch aktive Masse des Katalysators vor der Reduktion mit Wasserstoff 0,1 bis 5 Gew.-% sauerstoffhaltige Verbindungen des Molybdäns, berechnet als MoO₃, enthält.

3. Verfahren nach Anspruch 1, dadurch gekennzeichnet, dass die katalytisch aktive Masse des Katalysators vor der Reduktion mit Wasserstoff
22 bis 65 Gew.-% sauerstoffhaltige Verbindungen des Zirkoniums, berechnet als ZrO₂,
5 bis 25 Gew.-% sauerstoffhaltige Verbindungen des Kupfers, berechnet als CuO,
29,7 bis 70 Gew.-% sauerstoffhaltige Verbindungen des Nickels, berechnet als NiO,
0,3 bis 3,5 Gew.-% sauerstoffhaltige Verbindungen des Molybdäns, berechnet als MoO₃,
und 0 bis 10 Gew.-% sauerstoffhaltige Verbindungen des Aluminiums, berechnet als Al₂O₃, enthält.

4. Verfahren nach Anspruch 1, dadurch gekennzeichnet, dass die katalytisch aktive Masse des Katalysators vor der Reduktion mit Wasserstoff
25 bis 49,7 Gew.-% sauerstoffhaltige Verbindungen des Zirkoniums, berechnet als ZrO₂,
10 bis 25 Gew.-% sauerstoffhaltige Verbindungen des Kupfers, berechnet als CuO,
40 bis 60 Gew.-% sauerstoffhaltige Verbindungen des Nickels, berechnet als NiO,
0,3 bis 3,5 Gew.-% sauerstoffhaltige Verbindungen des Molybdäns, berechnet als MoO₃,
und 0 bis 5 Gew.-% sauerstoffhaltige Verbindungen des Aluminiums, berechnet als Al₂O₃, enthält.

5. Verfahren nach den Ansprüchen 1 bis 4, dadurch gekennzeichnet, dass man die Umsetzung bei Drücken von 0,1 bis 40 MPa durchführt.

6. Verfahren nach den Ansprüchen 1 bis 5, dadurch gekennzeichnet, dass man die Umsetzung bei Temperaturen von 80 bis 250°C durchführt.

7. Verfahren nach den Ansprüchen 1 bis 6, dadurch gekennzeichnet, dass man die Umsetzung bei Temperaturen von 80 bis 155°C durchführt.

8. Verfahren nach den Ansprüchen 1 bis 7, dadurch gekennzeichnet, dass man den Katalysator in Form von Formkörpern einsetzt.

9. Verfahren nach den Ansprüchen 1 bis 8 zur Herstellung von Aminen der Formel I 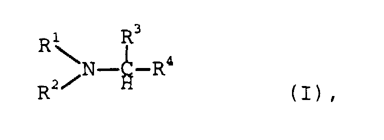 in der
R¹, R² Wasserstoff, C₁₋₂₀-Alkyl, C₃₋₁₂-Cycloalkyl, Aryl, C₇₋₂₀-Aralkyl und C₇₋₂₀-Alkylaryl oder gemeinsam (CH₂)ⱼ-X-(CH₂)ₖ,
R³, R⁴ Wasserstoff, Alkyl, Cycloalkyl, Hydroxyalkyl, Aminoalkyl, Alkanolaminoalkyl, Alkoxyalkyl, Dialkylaminoalkyl, Alkylaminoalkyl, R⁵-(OCR⁶R⁷CR⁸R⁹)ₙ-(OCR⁶R⁷), Aryl, Heteroaryl, Aralkyl, Heteroarylalkyl, Alkylaryl, Alkylheteroaryl und Y-(CH₂)ₘ-NR⁵-(CH₂)_{q} oder gemeinsam (CH₂)ₗ-X-(CH₂)ₘ oder
R² und R⁴ gemeinsam (CH₂)ₗ-X-(CH₂)ₘ,
R⁵, R¹⁰ Wasserstoff, C₁₋₄-Alkyl, C₇₋₄₀-Alkylphenyl,
R⁶, R⁷, R⁸, R⁹ Wasserstoff, Methyl oder Ethyl,
X CH₂, CHR⁵, Sauerstoff oder NR⁵,
Y N(R¹⁰)₂, Hydroxy, C₂₋₂₀-Alkylaminoalkyl oder C₃₋₂₀-Dialkylaminoalkyl,
n eine ganze Zahl von 1 bis 30 und
j, k, l, m, q eine ganze Zahl von 1 bis 4
bedeuten, durch Umsetzung von Aldehyden oder Ketonen der Formel II bzw. III 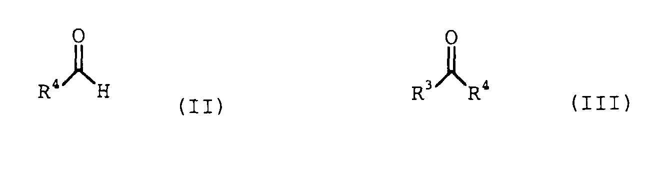 mit Stickstoffverbindungen der Formel IV
